# EUROPEAN PATENT APPLICATION

(11) **EP 1 880 721 A1**
(43) Date of publication of application: **23.01.2008**
(21) Application number: 06745757.2
(22) Date of filing: 27.04.2006
(51) Int. Cl.: A61K 31/409, A61K 31/7135, A61K 47/34, A61K 47/36, A61K 47/42, A61P 9/00, A61P 9/10

(54) **AGENT FOR IMPROVING CIRCULATORY DISORDER**

(30) Priority: 12.05.2005 JP 2005140282
(71) Applicant: NIPRO CORPORATION, Kita-ku, Osaka-shi, Osaka-fu, 531-8510 (JP)
(72) Inventor: Hirotsu, Ichiro, Kita-ku, Osaka-shi Osaka 5318510 (JP); Ohkawa, Yuki, Kita-ku, Osaka-shi Osaka 5318510 (JP); Nozako, Masanori, Kita-ku, Osaka-shi Osaka 5318510 (JP); Suzuki, Saori, Kita-ku, Osaka-shi Osaka 5318510 (JP); Kuwata, Yukiko, Kita-ku, Osaka-shi Osaka 5318510 (JP); Sato, Makoto, Kita-ku, Osaka-shi Osaka 5318510 (JP)
(74) Representative: Albrecht, Thomas
(86) International application number: PCT/JP2006/308829
(87) International publication number: WO 2006/120915

(57) **Abstract**

Provided is a pharmaceutical agent capable of efficiently improving blood circulation in a region where a circulatory disorder occurs and fully preventing or improving tissue damage as a preventive or therapeutic agent for circulatory disorders in general. As a result of the study of pharmacological effects of a compound in which a porphyrin metal complex is clathrated in albumin, it was found that the compound efficiently improves decreases in blood flow in circulatory disorders and fully prevents or improves tissue damage, thus the invention was achieved. That is, the invention is directed to an agent for improving a circulatory disorder including a compound in which a porphyrin metal complex is clathrated in albumin.

## Description

### Technical Field

The present invention relates to an agent that is used in the medical field for improving a blood circulatory disorder in a living body. More specifically, the present invention relates to an agent that increases blood flow to prevent or improve tissue damage for a circulatory disorders due to a decrease in blood flow, such as a deterioration of a cardiac function, systemic persistent hypertension, hypotension, circulatory shock, and local hyperemia, congestion, oligemia or ischemia, infarct, or bleeding.

### Background Art

Blood circulatory disorders are broadly classified into central blood circulatory disorders and peripheral blood circulatory disorders, and the peripheral blood circulatory disorders are further classified into systemic blood circulatory disorders and local blood circulatory disorders. The central blood circulatory disorders are caused primarily by deteriorated cardiac function and include acute and chronic left ventricular dysfunction and right ventricular dysfunction. A systemic peripheral blood circulatory disorder is a peripheral circulatory disorder which arises in the entirety of the blood circulating system, but not of cardiac primary cause, and is caused from persistent hypertension, hypotension, circulatory shock, and the like. The local peripheral blood circulatory disorder is a disorder involved in local hyperemia, congestion, oligemia or ischemia, infarct, bleeding, etc.

As prophylactic or therapeutic agents for such circulatory disorders, vasodilators, thrombolytics, and the like have been used, which serve as therapeutic agents for clinical conditions that cause the circulatory disorders and as an agent for directly improving the circulatory disorders. However, reperfusion tissue damage and the like are caused by active oxygen after a decrease in systemic blood pressure and thrombolysis, and therefore there have never been developed an agent capable of effectively improving blood circulation at a region where a circulatory disorder occurs and capable of fully preventing or improving tissue damage.

On the other hand, various artificial oxygen infusions have been studied for the purposes of use as a resuscitative fluid for hemorrhagic shock (erythrocyte substitute), a solution for oxygen supply to an ischemic region in cardiac infarction and the like, a perfusion fluid or a preservation solution for transplant organs, a supplying solution for an extracorporeal circuit such as a artificial heart-lung machine, an oxygen supply solution for cultured cells of regenerated tissue, a sensitizer for cancer therapy, etc. A compound in which a porphyrin metal complex is clathrated in albumin has been studied as one example of the artificial oxygen infusions (see Patent Document 1, for example).
Patent Document 1: JP 2004-10495 A

### Disclosure of the Invention

### Problems to be solved by the Invention

The present invention is intended to provide, as a prophylactic or therapeutic agent for the above-mentioned circulatory disorders in general, an agent capable of effectively improving blood circulation at a region where a circulatory disorder occurs to fully prevent or improve tissue damages.

### Means for Solving the Problems

The inventors of the present invention have studied pharmacological effects of a compound in which a porphyrin metal complex is clathrated in an albumin, and as a result, they have discovered that the compound effectively improves a decrease in blood flow due to a circulatory disorder to sufficiently prevent or improve tissue damage, thereby achieving the present invention.

The inventors of the present invention have examined a compound in which a porphyrin metal complex is clathrated in albumin using a plurality of animal models of decreasedblood flow or ischemia, and as a result, they have confirmed that the compound shows sufficient effects of improving blood flow and suppressing tissue damage in all of the models.

That is to say, the present invention relates to:
(1) an agent for improving a circulatory disorder, including a compound in which a porphyrinmetal complex is clathrated in albumin;
(2) an agent for improving a circulatory disorder according to Item (1), in which the porphyrin metal complex is an iron complex;
(3) an agent for improving a circulatory disorder according to Item (1), in which the iron is in a divalent state;
(4) an agent for improving a circulatory disorder according to Item (1), in which the albumin is one of human serum albumin and recombinant human serum albumin;
(5) an agent for improving a circulatory disorder according to Item (1) , in which the albumin is modified with a polymer compound; and
(6) an agent for improving a circulatory disorder according to Item (1) , in which the polymer compound is at least one selected from the group consisting of polyethylene glycol, dextran, and polyamino acid.

### Effects of the Invention

An, agent for improving a circulatory disorder of the present invention has an effect of increasing blood flow volume at a region where a circulatory disorder occurs at the time of a decrease in blood flow volume due to bleeding, vascular stenosis, obstruction, etc. In addition, the effect of increasing blood flow volume leads to an effect of improving tissue damage.

### Brief Description of the Drawings

Fig. 1 shows images created by a Laser Doppler blood flow imaging device for comparison of effects on decreases in blood flow volumes at the lower limbs in blood-removed rats in the cases of (1) administration of 5% rHSA and (2) administration of rHSA-FecycP.

Fig. 2 shows graphs for comparison of effects of improving ischemic cardiac dysfunctions in rat isolated hearts in the cases of (1) normal control, (2) ischemia control by slow perfusion, (3) ischemia + 5% rHSA administration, and (4) ischemia + rHSA-FecycP administration.

### Best Mode for carrying out the Invention

A porphyrin metal complex used in the present invention is a tetraphenylporphyrin metal complex having 2-position side chains in which a central metal is coordinated to a porphyrin derivative. The porphyrin is a macrocyclic compound in which four pyrrole rings are alternately bound to four methine groups at α-positions, and a derivative thereof.

The porphyrinmetal complex used in the present invention is preferably a complex represented by the following chemical formula (1) . 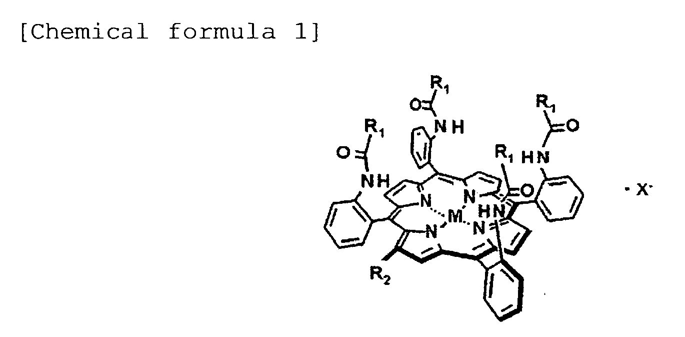 (In chemical formula (1), R₁ represents a chain or alicyclic hydrocarbon group which may have a substituent, R₂ represents a substituent represented by the following chemical formula (3), M represents a transition metal atom belonging to Groups 6 to 10 of the periodic table or a metal ion thereof, X⁻ represents a halogen ion, and the number of X is obtained by subtracting two from the valence of the metal ion.)

In the porphyrin metal complex of the present invention, R₁ in the above chemical formula (1) is preferably a substituent including a cyclohexanoyl group, and for example, a porphyrin metal complex represented by the following chemical formula (2) is preferable. 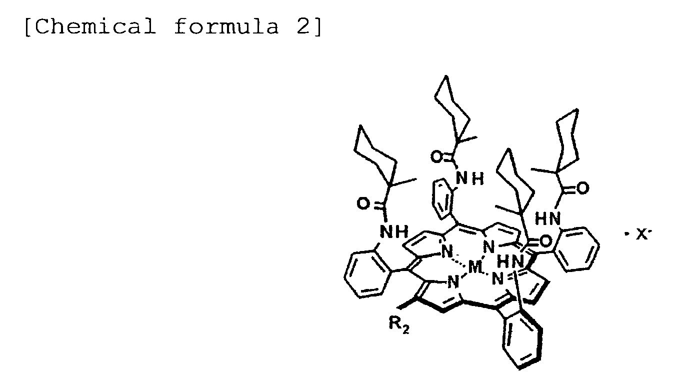 (In chemical formula (2), R₂ represents a substituent represented by the following chemical formula (3), M represents a transition metal atom belonging to Groups 6 to 10 of the periodic table or a metal ion thereof, X⁻ represents a halogen ion, and the number of X⁻ is obtained by subtracting two from the valence of the metal ion.)

Meanwhile, in the chemical formula (1) or (2), examples of the substituent at position 2 (R₂) include one represented by the following chemical formula (3). 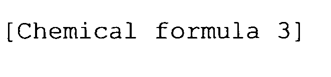 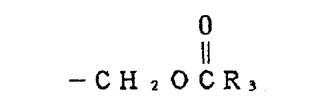 (In chemical formula (3), R₃ represents a C₁-C₁₈ alkyl group or a group represented by the following chemical formula (4).) 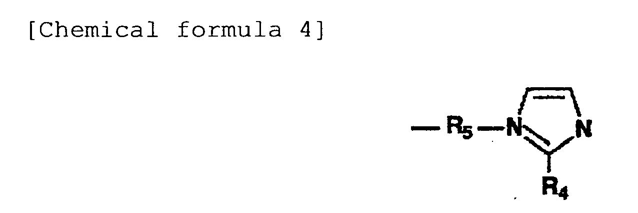 (In chemical formula (4) , R₄ represents a group which does not inhibit coordination to a central metal of imidazole to which R₄ binds, and R₅ represents an alkylene group.)

In the chemical formula (4), R₄ is preferably hydrogen or a lower alkyl group such as a methyl, ethyl, or propyl group, and R₅ is preferably a C₁-C₁₀ alkylene group.

The tetraphenylporphyrin metal complex of the present invention represented by the chemical formula (1) is preferably a complex in which a base is coordinated to a central metal (M). Meanwhile, the complex is preferably an imidazole derivative in which the substituent at position 2 itself is coordinated to a central metal as an axis base, and examples thereof include a porphyrin metal complex represented by the following chemical formula (5). 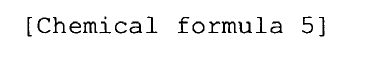 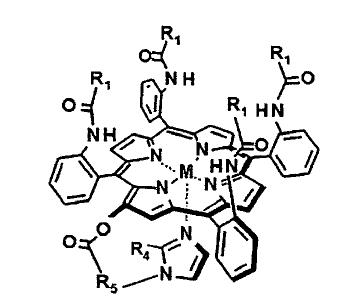 (In chemical formula (5), R₁ represents a chain or alicyclic hydrocarbon group which may have a substituent, R₄ represents a lower alkyl group, and R₅ represents a C₁-C₁₀ alkylene group. M represents iron, cobalt, or chromium, or an ion thereof.)

The central metal in the porphyrin metal complex of the present invention (for example, M in the chemical formula (1)) is a transition metal atom belonging to Groups 6 to 10 of the periodic table and may be a metal ion thereof. Of them, the metal is preferably iron, cobalt, or chromium, and more preferably divalent iron (iron (II)), trivalent iron (iron (III)), or divalent cobalt (cobalt (II)). Still more preferable is divalent iron.

Of the porphyrin metal complexes, a particularly preferred example thereof is a 2-[8-(2-methyl-1-imidazolyl)octanoyloxymethyl]-5,10,15,20-tetr akis{[α,α,α,α-o-(1-methylcyclohexanoyl)]phenyl}porphinatoiron( II) complex represented by the following chemical formula (6).

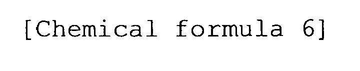

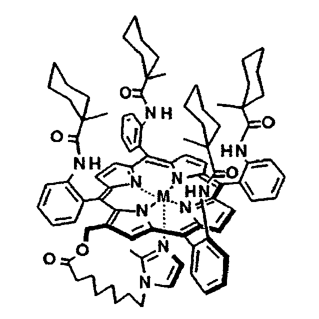

Examples of the albumin and porphyrin iron complex used in the present invention include a complex (rHSA-Fecyc3P) in which iron is coordinated to the center of 5,10,15-tris(α,α,α-o-cyclohexanamidephenyl)-20-mono[β-o-6-{N-( 2-methyl-1-imidazolyl)}hexanamidephenyl] porphyrin represented by the following chemical formula (7), which is designed to increase the yield by symplifing a synthetic process of 2-[8(2-methyl-1-imidazolyl)octanoyloxymethyl]-5,10,15,20-tetra kis{[α,α,α,α-o-(1-methylcyclohexanoyl)]phenyl}porphinatoiron(I I) complex (FecycP) represented by the chemical formula (6) to omit use of a harmful substance in a synthetic procedure.

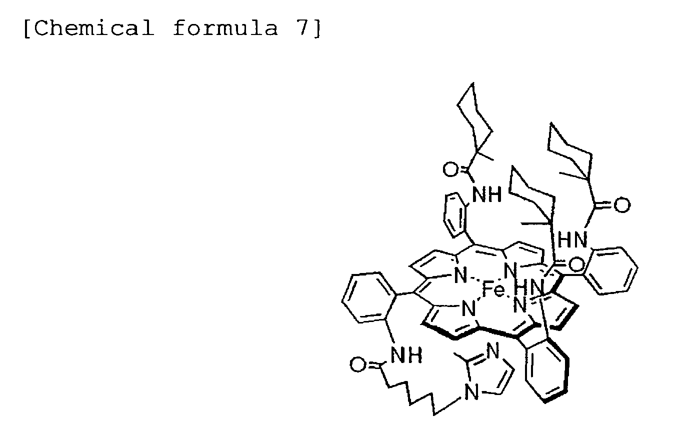

A compound of the present invention, in which a porphyrin metal complex is clathrated in albumin, is obtained by embedding and fixing (including) a porphyrin metal complex in a hydrophobic region formed inside albumin.

The number of porphyrin metal complexes bound to 1 mole of albumin (such as human serum albumin) is generally about 1 to 8. The number of porphyrin metal complexes included in or bound to 1 mole of albumin can be determined by producing a Scatchard plot (C. J. Halfinan, T. Nishida, Biochemistry, 11, p.3493 (1972)), for example.

Examples of albumin to be used in the present invention include human serum albumin, recombinant human serum albumin, and bovine serum albumin, and the origin of albumin is not particularly limited. In view of application to humans, human serum albumin or human serum albumin produced through gene recombination technology (hereinafter, referred to as recombinant human serum albumin) is preferred, and recombinant human serum albumin is particularly preferred.

Recently, high purity recombinant human serum albumin having a completely identical structure, composition, and physicochemical properties to those of human serum albumin has been developed owing to the development of gene recombination technology (see K. Kobayashi et al., Therapeutic Apheresis, Second Edition, p.257-262 (1998)). An inclusion compound porphyrin metal complex-recombinant human serum albumin obtained by including a substituted porphyrin metal complex in recombinant human serum albumin can be provided as a totally synthetic system, and thus can be produced on an industrial scale with relative ease. Further, as albumin is a plasma protein, it is excellent in application to a living body.

The albumin used in the present invention is preferably modified with a polymer compound. The polymer compound is not particularly limited as long as it is a polymer compound that can be used for medical purposes and can modify albumin. Examples thereof include polyethylene glycol, dextran, and polyamino acid, and preferable is polyethylene glycol.

Polyethylene glycol is a polymer that has been widely used for molecular modification in peptide drugs because polyethylene glycol was used, for example, in 1997 by Abuchowski et al. (see A. Abuchowski et al . , Journal of Biological Chemistry, 252, 3578-3581 (1977) and Journal of Biological Chemistry, 252, 3582-3586 (1977)), and many examples of studies carried out for the purposes of extension of half-life in vivo and a decrease in antigenicity (see Y. Kawasaki et al., Journal of Pharmacology and Experimental Therapeutics, 216, 410-414 (1981), N. Katre et al., Proceedings of the National Academy of Sciences of the United States of America, 84, 1487-1491 (1987), and MJ. Knauf et al., Journal of Biological Chemistry, 263, 15064-15070 (1988)) have been reported.

The agent for improving a circulatory disorder of the present invention is an agent containing a compound in which a porphyrin metal complex is clathrated in albumin, and various pharmaceutical compositions including a stabilizer and the like in addition to the agent are also within the scope of the present invention.

The dosage form of the agent for improving a circulatory disorder of the present invention may be any one of the various dosage forms for medical use, and an injection is preferable. In the case of producing the agent of the present invention as an injection, an appropriate solvent, and if necessary, pH adjuster, buffer, stabilizer, suspension, solubilizer, carrier, etc. may be added to prepare an injection in accordance with a conventional method.

The agent for improving a circulatory disorder of the present invention is used for treatment and prevention of a circulatory disorder caused by variousdiseases/symptoms. Examples of the various diseases/symptoms include: diseases such as high blood pressure, hyperlipemia, and diabetes; ischemic symptoms caused by vascular obstructions due to thrombus or the like, and acute or chronic bleeding; reperfusion damage after ischemia; and others such as organ transplantation. Examples of the circulatory disorder caused by these diseases/symptoms include ischemic brain diseases (cerebral infarction, transient cerebral ischemia attack, blood clots, and cerebral embolisms), cerebral infarction sequelae, cerebral vasoconstriction, ischemic heart disease (cardiac infarction), angina, thromboangiitis obliterans (Buerger's disease), arteriosclerosis obliterans, thrombophlebitis, Raynaud's disease and Raynaud's syndrome, acrocyanosis, chilblain/frostbite, acroparesthesia, intermittent claudication, spontaneous gangrene, Meniere's disease, Meniere's syndrome and vertigo, chronic artery obstruction, ischemic pancreatitis, ischemic optic neuropathy , and ischemic colitis.

The agent for improving a circulatory disorder of the present invention is used for organs such as the brain, heart, lung, liver, kidney, pancreas, spleen, intestines, skeletal muscle, bone marrow, nerve, retina, and blood vessels.

Examples of an administration method in the case where the agent for improving a circulatory disorder of the present invention is administered as an injection include, for example, intravenous administration, intraarterial administration, intraportal administration, and direct intralesional administration.

The dose of the agent for improving a circulatory disorder of the present invention depends on the age, weight, sex, administration method, symptom of a patient, but in general, the agent is parenterally administered (for example, by injection or continuous drip) in an amount within a range of from about 1 to 2,500 mL (for example, 4 mg to 11 g as FecycP), preferably about 1 to 1,000 mL (for example, 4 mg to 4 g as FecycP) per adult per day.

Hereinafter, the present invention will be described in more detail by using examples, but the present invention is not limited thereto.

### Example 1

2-[8-(2-methyl-1-imidazolyl)octanoyloxymethyl]-5,10,15,20 -tetrakis{[α,α,α,α-o-(1-methylcyclohexanoylamino)]phenyl}porph inatoiron (II) complex used in the following Examples can be produced through a production method described in T. Komatsu et al., Bioconjugate Chemistry 13th Edition, p. 397-402, (2002).

### (Production Example 1) Production of porphyrin iron complex-albumin inclusion compound

To 1.5 L of a solution of 1.07 mmoL 2-[8(2-methyl-1-imidazolyl)octanoyloxymethyl]-5,10,15,20-tetra kis{[α,α,α,α-o-(1-methylcyclohexanoylamino)]phenyl}porphinatoi ron complex in ethanol was added 1.5 L of an aqueous solution of 0.6 M L-ascorbic acid in a carbon monoxide atmosphere to reduce the complex, and the solution was added to 6.5 L of an aqueous phosphate buffer solution (pH 7.4, 1/30 mM) containing 0.27 mmoL recombinant human serum albumin (hereinafter, abbreviated as rHSA), followed by stirring. To the mixed solution was added 60 L of an aqueous phosphate buffer solution (pH 7.4, 1/30 mM) while constant volume ultrafiltration dialysis was performed using an ultrafiltration device (a ultrafiltration membrane manufactured by Millipore Corporation:ultrafiltration molecular weight of 30,000), to thereby remove ethanol in the mixed solution. The mixed solution was concentrated to 300 mL, and the concentration of rHSA was adjusted with a phosphate buffer (pH 7.4, 1/30 mM) so that the final concentration reached 0.75 mM (5 w/v%), to thereby yield a desired dispersion of a porphyrin iron complex-albumin inclusion compound (hereinafter, abbreviated as rHSA-FecycP).

rHSA-FecycP was exposed to light using a 500 W halogen lamp for 20 minutes in a stream of an oxygen gas (100%) and then used in Experimental Examples 1 and 2. Note that the rHSA was purchased from Baifa Corporation (25 w/v% formulation).

### (Experimental Example 1)

### Effect on rat model with hemorrhagic blood flow decrease

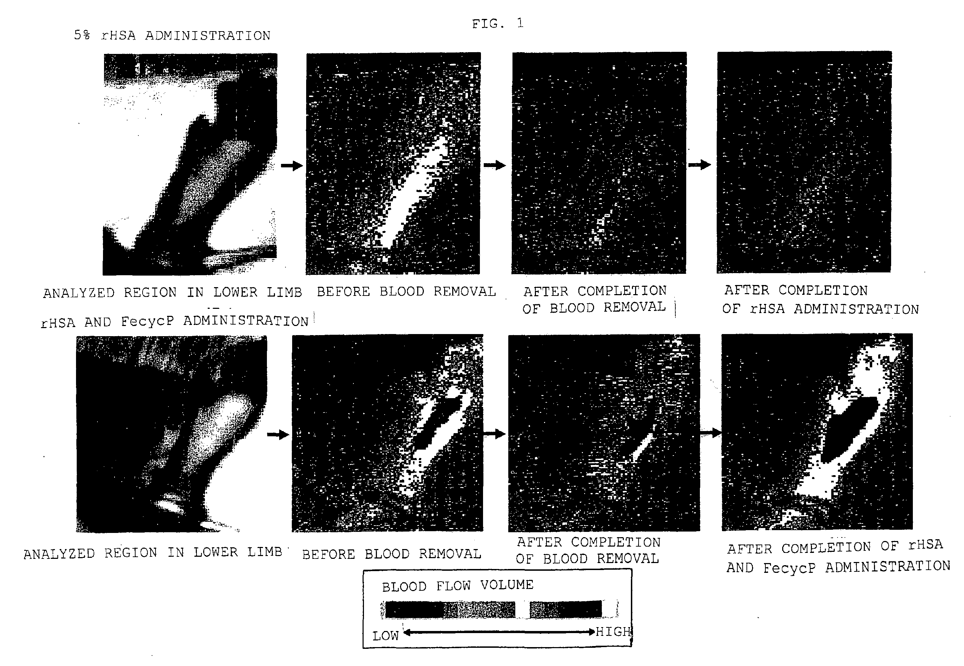

For each Wister male rat (8-week-old), blood removal was performed under sevoflurane inhalation anesthesia (introduction 2.0%, maintenance 1.5%) to decrease the blood flow volume. Removal of 2 mL of blood from the right common carotid artery and administration of 2 mL of 5% rHSA from the right femoral vein were repeated nine times to exchange about 70% of the total blood volume with 5% rHSA, and ten minutes later, 30% of the total blood volume was removed from the right common carotid artery. After completion of blood removal, rHSA-FecycP or 5% rHSA as a control was rapidly administered to the right femoral vein in an amount of 30% of the total blood volume. Note that the blood removal and administration were performed at a rate of 1 mL/minute. Before blood removal, immediately after completion of blood removal, and 25 minutes after completion of administration, blood flow volumes at the lower limb were noninvasively measured using a Laser Doppler blood flow imaging device (manufactured by Monte System Corporation, MoorLDI-LKII). Images were created from the results and are shown in Fig. 1.

The blood flow volume of the rat decreased after completion of blood removal, and even in the case of 5% rHSA administration, the blood flow volume did not recover and further decreased. On the other hand, in the case of rHSA-FecycP, the blood flow volume decreased by blood removal recovered to a level approximately equal to that before blood removal. Therefore, rHSA-FecycP was found to have an effect of increasing and improving a blood volume for hemorrhagic blood flow decrease.

### (Experimental Example 2)

### Effect on rat model with ischemic cerebral blood flow decrease

An experiment was performed on an effect of improving a decrease in improvement of a cerebral blood flow of a rat with obstructions in the bilateral common carotid arteries in the case of administration of rHSA-FecycP.

For each Wister male rat (10-week-old), the bilateral common carotid arteries were ligated under pentobarbital sodium anesthesia (50 mg/kg, intraperitoneal administration) to decrease the cerebral blood flow. Ten minutes after ligation of the bilateral common carotid arteries, rHSA-FecycP was administered into the tail vein in an amount of 10 mL/kg. Before ligation of the bilateral common carotid arteries, immediately after ligation, and five minutes after administration of rHSA-FecycP, the blood flow volumes in the brain cortex were noninvasively measured using a Laser Doppler blood flow imaging device (manufactured by Monte System Corporation, MoorLDI-LKII) . Images created from the results were quantified and are shown in Table 1, where a value before ligation is defined as 100%.

**[Table 1]**

| Rat No. | Blood flow volume in cerebral cortex (% value based on a value before ligation defined as 100) | | |
|---|---|---|---|
| | Before ligation of common carotid artery | after Immediately after ligation | Five minutes after administration of 10 mL/kg rHSA-FecycP |
| 1 | 100 | 55.4 | 63.7 |
| 2 | 100 | 73.7 | 82.8 |

The results shown in Table 1 revealed that the blood flow volumes in the cerebral cortices decreased to 55.4% and 73.7% immediately after ligation compared to those before ligation of common carotid arteries (100%), and the blood flow volumes increased to 63.7% and 82.8%, respectively, by intravenous administration of 10 mL/kg rHSA-FecycP. Therefore, rHSA-FecycP was found to increase and improve a blood flow volume for ischemic cerebral blood flow decrease.

### (Experimental Example 3)

### Effect on ischemic cerebral dysfunction due to decrease in cerebral blood flow

An experiment was performed on an effect of improving ischemic cerebral dysfunction caused by a decrease in a cerebral blood flow of a rat with obstructions in the bilateral common carotid arteries in the case of administration of rHSA-FecycP.

For each Fischer male rat (8-week-old), the bilateral common carotid arteries were ligated under ether light anesthesia to induce cerebral ischemia. rHSA-FecycP (1 and 3 mL/kg) or 5% rHSA (control, 3 mL/kg) was administered into the tail vein immediately after obstructions in the bilateral common carotid arteries, and observations were performed on the presence or absence of manifestation of ischemic attacks (paroxysmal jumping and convulsion) during 3.5 hours after completion of the obstructions and on the life or death during 24 hours after completion of the obstructions during wakefulness. The manifestation rate of the ischemic attacks (the number of manifestation cases/the number of use cases x 100 (%)) and death rate (the number of death cases/the number of use cases x 100 (%)) were used as indexes to examine the effect of the drug. The results are shown in Table 2.

**[Table 2]**

| Compound | Dose (mL/kg) | Number of cases | Manifestation rate of ischemic attacks (%) | Death rate(%) |
|---|---|---|---|---|
| 5% rHSA | 3 | 5 | 80 | 100 |
| rHSA-FecycP | 1 | 6 | 33 | 83 |
| | 3 | 6 | 33 | 67 |

In the case of administration of 3 mL/kg rHSA, the manifestation rate of the ischemic attacks was found to be 80%, while in the cases of administration of 1 and 3 mL/kg rHSA-FecycP, the respective rates were found to decrease to 33%. Meanwhile, in the case of administration of 3 mL,/kg rHSA, the death rate was found to be 100%, while in the cases of administration of 1 and 3 mL/kg rHSA-FecycP, the rates were found to decrease to 87 and 67%, respectively. Therefore, rHSA-FecycP was found to have an effect of improving ischemic cerebral dysfunction.

### (Experimental Example 4)

### Effect of improving ischemic cerebral dysfunction by slow perfusion

The heart of each Wister male rat (8- to 10-week-old) was removed under pentobarbital sodium anesthesia, and a cannula was inserted into the aorta in accordance with the Langendorff method to perfuse with a nutrition solution. In order to measure a heart function, i.e., the force of heart contraction, a balloon containing physiological saline was inserted into the left ventricle, and the resultant pressure waveform was analyzed to determine the end-diastolic pressure and maximum rising rate of the left ventricular pressure. As a nutrition solution, a Krebs-Henseleit solution (NaCl 118 mM, KCl 4.7 mM, KH₂PO₄ 1.2 mM, MgSO₄ 1.2 mM, CaCl₂ 2.5 mM, NaHCO₃ 25 mM, glucose 11 mM, pH 7.4) aerated with a 95% O₂-5 % CO₂ mixed gas and incubated at 37 °C was used, and the solution was perfused at a flow rate of 20 mL/min for 20 minutes to stabilize the state. Myocardial ischemia was induced by performing slow perfusion at a flow rate decreased to 1/10 (2 mL/min) for 60 minutes. For a 5% rHSA group (ischemia + 5% rHSA group, n = 7) and rHSA-FecycP group (ischemia + rHSA-FecycP group, n = 7), 5% rHSA and rHSA-FecycP were separately applied continuously to the perfusion solution from a side tube of the cannula inserted into the aorta at a rate of 0.2 mL/min for 60 minutes, simultaneously with the flow rate being decreased. For an ischemia control group (n = 7), no drug solution was applied from the side tube.

After completion of slow perfusion, the flow rate recovered to 20 mL/min again, and perfusion was continued for 60 minutes. Pacing of the heart (heart rate 330 beats/min) was performed using an electrostimulator.

For each experiment group, at the respective time points from the start of slow perfusion to 120 minutes later, the end-diastolic pressure and maximum rising rate of the left ventricular pressure were measured.

Note that, for a normal control group (n = 7), which was subjected to perfusion at a rate of 20 mL/min during the entire term of the experiment, the end-diastolic pressure and maximum rising rate of the left ventricular pressure were measured for comparison in the same way as above.

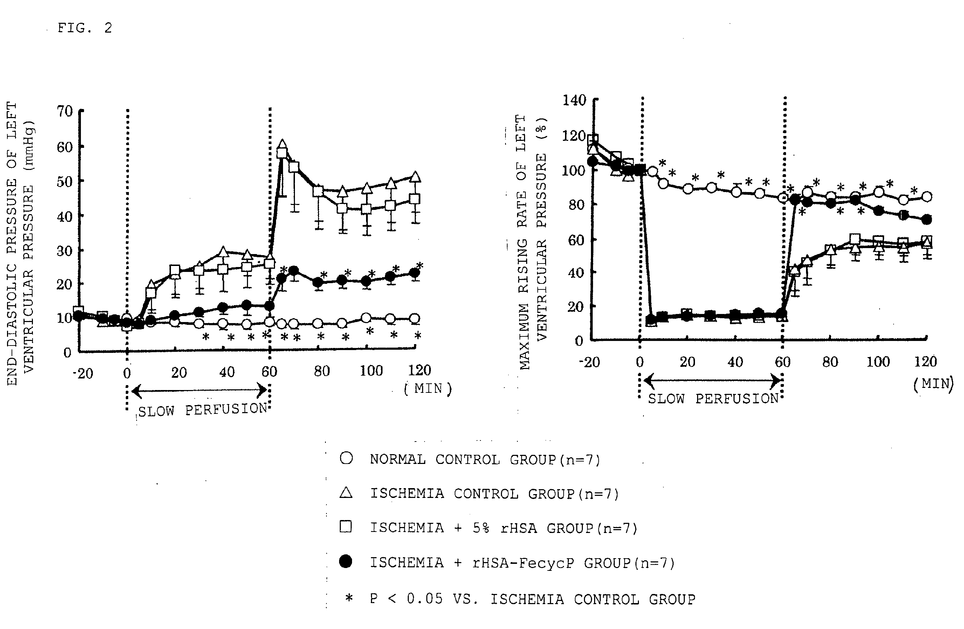

The maximum rising rate of the left ventricular pressure is represented based on a value at the start of slow perfusion defined as 100%. Meanwhile, for each group, the end-diastolic pressure and maximum rising rate of the left ventricular pressure are represented as mean ± standard error, and the Dunnett's test and Steel's test were performed as significant difference tests (in both the tests, significant levels were 5%). The results are shown in the graphs in Fig. 2.

The end-diastolic pressure of the left ventricular pressure increased with the decrease in the force of heart contraction, but in the normal control group, the pressure was approximately constant (7.0 to 11.9 mmHg) during the experiment. In the case of the ischemia control group, the pressure gradually increased after the start of ischemia by slow perfusion, and 60 minutes after ischemia when the flow rate recovered to a level equal to that before ischemia, the pressure further significantly increased, resulting in ischemia reperfusion damage. In the case of the 5% rHSA group, the pressure changed almost in the same way as the ischemia control group. On the other hand, in the case of the rHSA-FecycP group, the pressure was maintained at a lower level than that of the ischemia control group during the slow perfusion ischemia period. Although slightly increased when the flow rate recovered to a level before ischemia, the pressure significantly decreased compared to the ischemia control group.

The maximum rising rate of the left ventricular pressure decreases with the decrease in the force of heart contraction, but in the case of the normal control group, the rate gradually decreased with time and decreased by about 30% at a maximum. In the case of the ischemia control group, the rate significantly decreased after the start of ischemia by slow perfusion and increased 60 minutes after ischemia when the flow rate recovered to a level before ischemia. However, the rate recovered only to about 60%. In the case of the 5% rHSA group, the rate changed almost in the same way as the ischemia control group. On the other hand, in the case of the rHSA-FecycP group, the rate changed almost in the same way as the ischemia control group during the slow perfusion ischemia period. However, five minutes after recovering the flow rate to a level before ischemia, the rate increased to a level approximately equal to that of the normal control group. 5, 20, and 30 minutes after completion of slow perfusion, the rates were significantly higher than the rate of the ischemia control group. Therefore, rHSA-FecycP was found to suppress cardiac dysfunctions induced by cardiac ischemia and ischemia reperfusion.

As described above, since rHSA-FecycP increase a flow blood volume decreased by bleeding, vascular obstruction, vascular stenosis, or the like, and improve functional damages in various tissues, it is obvious that the agent of the present invention is effective as an agent for improving a circulatory disorder.

An inclusion compound of the present invention can be used for various medical applications as an agent for improving a circulatory disorder, and applied to circulatory disorders due to various diseases/symptoms.

## Claims

1. An agent for improving a circulatory disorder, comprising:
a compound in which a porphyrin metal complex is clathrated in albumin.

2. An agent for improving a circulatory disorder according to Claim 1, wherein the porphyrin metal complex is an iron complex.

3. An agent for improving a circulatory disorder according to Claim 1, wherein the iron is in a divalent state.

4. An agent for improving a circulatory disorder according to Claim 1, wherein the albumin is human serum albumin or recombinant human serum albumin.

5. An agent for improving a circulatory disorder according to Claim 1, wherein the albumin is modified with a polymer compound.

6. An agent for improving a circulatory disorder according to Claim 1, wherein the polymer compound is at least one selected from the group consisting of polyethylene glycol, dextran, and polyamino acid.

7. An agent for improving a circulatory disorder according to Claim 1, wherein the porphyrin metal complex is a compound represented by chemical formula (1): 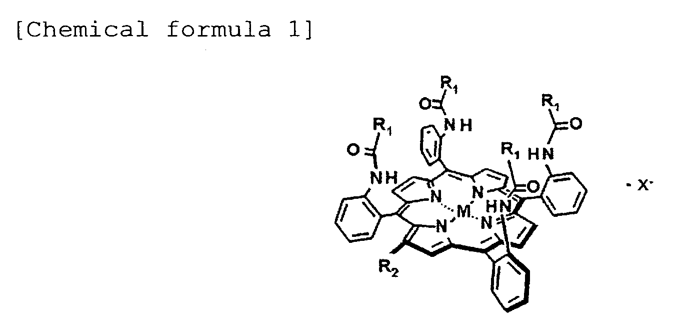 where R₁ represents a chain or alicyclic hydrocarbon group which may have a substituent, and R₂ represents a group represented by the following chemical formula (3); 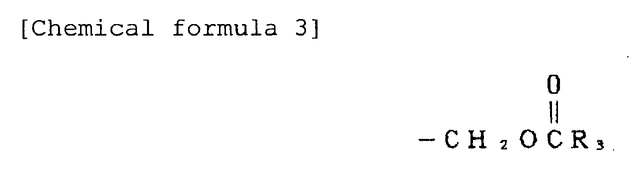 where R₃ represents a C₁-C₁₈ alkyl group or a group represented by chemical formula (4); 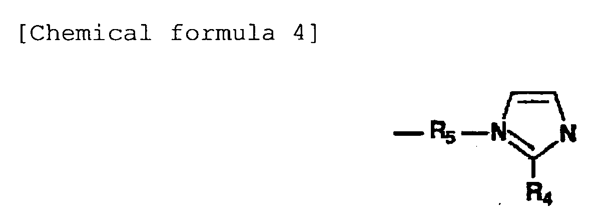 where R₄ represents a lower alkyl group, and R₅ is a C₁-C₁₀ alkylene group;
M represents a transition metal atom belonging to Groups 6 to 10 of the periodic table or a metal ion thereof, X⁻ represents a halogen ion, and the number of X⁻ is obtained by subtracting two from the valence of the metal ion.

8. An agent for improving a circulatory disorder according to Claim 1, wherein the porphyrin metal complex is a compound represented by chemical formula (2): 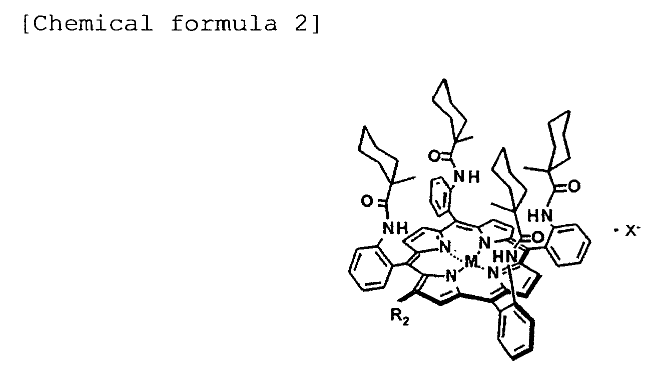 where R₂ represents a group represented by the following chemical formula (3); 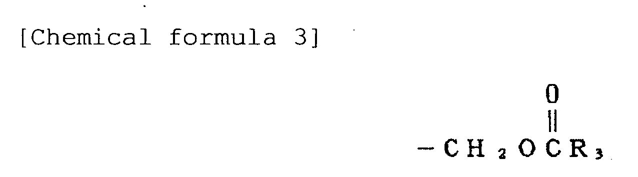 where R₃ represents a C₁-C₁₈ alkyl group or a group represented by chemical formula (4); 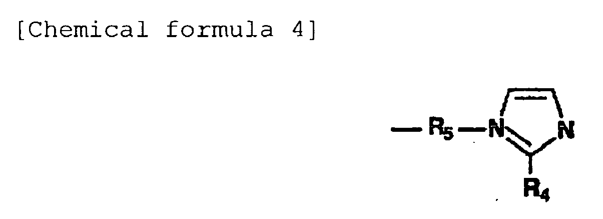 where R₄ represents a lower alkyl group, and R₅ is a C₁-C₁₀ alkylene group;
M represents a transition metal atom belonging to Groups 6 to 10 of the periodic table or a metal ion thereof, X⁻ represents a halogen ion, and the number of X⁻ is obtained by subtracting two from the valence of the metal ion.

9. An agent for improving a circulatory disorder according to Claim 1, wherein the porphyrin metal complex is a compound represented by chemical formula (5): 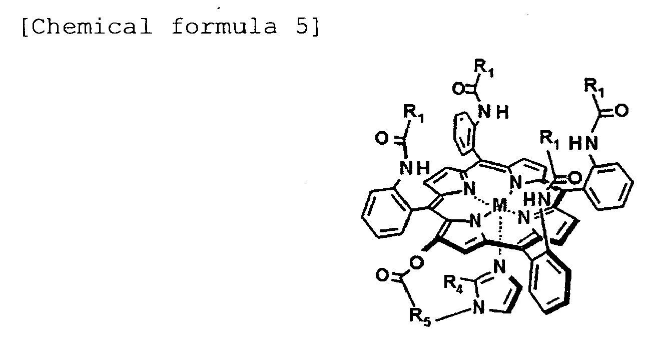 where R₁ represents a chain or alicyclic hydrocarbon group which may have a substituent, R₄ represents a lower alkyl group, and R₅ represents a C₁-C₁₀ alkylene group; M is iron, cobalt, or chromium, or an ion thereof.

10. An agent for improving a circulatory disorder according to Claim 7 or 8, wherein the metal atom or metal ion is iron, cobalt, or chromium, or an ion thereof.
